# FASCICULE DE BREVET EUROPEEN

(11) **EP 3 873 595 B1**
(45) Date de publication et mention de la délivrance du brevet: **25.02.2026**
(21) Numéro de dépôt: 19813115.3
(22) Date de dépôt: 25.10.2019
(51) Int. Cl.: A61N 5/06

(54) **DISPOSITIF POUR LE TRAITEMENT DE PATIENTS PAR PHOTOTHERAPIE**
VORRICHTUNG ZUR BEHANDLUNG VON PATIENTEN DURCH PHOTOTHERAPIE
DEVICE FOR TREATING PATIENTS BY PHOTOTHERAPY

(30) Priorité: 31.10.2018 FR 1860085
(43) Date de publication de la demande: 08.09.2021
(73) Titulaire: MDB Texinov, 38110 Saint Didier de la Tour (FR); Deltaval, 69006 Lyon (FR)
(72) Inventeur: BERGER, Sandra, 38490 FITILIEU (FR); DONGE, Charlotte, 38110 SAINT JEAN DE SOUDAIN (FR); PLAT, Anne, 01120 DAGNEUX (FR); TANKERE, Jacques, 01800 MEXIMIEUX (FR)
(74) Mandataire: Ipsilon
(86) Numéro de dépôt international: PCT/FR2019/052549
(87) Numéro de publication internationale: WO 2020/089550

(56) Documents cités:
- CN-U- 202 314 978
- FR-A1- 3 029 944
- US-A1- 2009 030 489
- US-A1- 2010 114 263
- US-A1- 2016 051 832

## Description

### DOMAINE DE L'INVENTION

L'invention concerne le domaine du traitement de patients par photothérapie. Plus particulièrement, l'invention vise le traitement de l'ictère de nouveau-nés ou d'enfants nés prématurément.

Elle vise notamment de traitement par photothérapie à la fois simple à mettre en œuvre, confinant la lumière émise par un tel dispositif aux zones du corps du patient devant être soumises au traitement, et en outre assurant une sécurité optimisée, notamment lorsque le patient est un nouveau-né ou un enfant en très bas âge.

### ETAT ANTERIEUR DE LA TECHNIQUE

L'ictère du nourrisson, se traduisant par une coloration jaune des téguments due à l'accumulation de bilirubine dans l'organisme, également mieux connu sous le terme jaunisse, est relativement répandu.

Différents traitements sont connus pour soigner cette maladie et notamment :
- pour un ictère léger, le traitement consiste à faire téter de manière abondante le nouveau-né afin de favoriser l'élimination de la bilirubine via les selles et les urines ;
- les traitements médicamenteux par la mise en œuvre d'inducteurs enzymatiques, et perfusion d'albumine afin de capter la bilirubine ;
- l'exsanguino-transfusion, c'est-à-dire le remplacement d'une partie importante du sang du nouveau-né ;
- la photothérapie, c'est-à-dire la soumission du nouveau-né à un rayonnement non ionisant, dont la longueur d'onde est typiquement comprise entre 420 et 490 nanomètres, ladite lumière dégradant la bilirubine.

Dans le domaine de la photothérapie, l'une des techniques classiquement utilisées consiste à positionner le nouveau-né dans un tunnel, sous des lampes émettant de la lumière bleue pour assurer un tel traitement par photothérapie, les yeux du nourrisson étant occultés par tous moyens appropriés, et les parties génitales protégées. La durée d'exposition du nourrisson dans ce tunnel peut varier de plusieurs heures d'affilée, ce pendant plusieurs jours selon le degré de gravité de la jaunisse.

Cette technique présente un certain nombre d'inconvénients, parmi lesquels on peut citer :
- la séparation pendant plusieurs heures par jour de la mère et de l'enfant, affectant le lien essentiel se développant entre ces derniers dès après la naissance ;
- la nécessité de l'hospitalisation de l'enfant en service pédiatrique et non en maternité, si l'ictère n'est détecté qu'après la sortie de la maternité ;
- la génération de chaleur inhérente aux lampes ou systèmes irradiants situés à l'intérieur de tels tunnels, susceptible d'entraîner des brûlures en particulier lors de la mise en œuvre de lampes halogènes, et dans des cas extrêmes l'hyperthermie voire le décès du nouveau-né, nécessitant d'associer à ces dispositifs des systèmes d'alarme de température et de ventilation pour dissiper la chaleur ;
- une exposition non uniforme du corps du nouveau-né, inhérente à la forme particulière de ces tunnels, notamment convexe ;
- l'interruption nécessaire de la durée d'exposition du nouveau-né, en raison des soins indispensables à apporter à l'enfant notamment en termes d'alimentation.

En raison de ces différents inconvénients, il a été proposé une autre technique consistant à mettre en œuvre des dispositifs textiles souples, permettant tout d'abord une réduction drastique des coûts de réalisation, et en outre d'optimiser de manière importante le confort, notamment du nourrisson.

On a par exemple décrit dans le document KR 2016 0143181, un dispositif se présentant sous la forme d'une couverture, dans laquelle un réseau de diodes LED reliées par des fils métalliques est intégré. En raison cependant de son mode de réalisation, ce dispositif demeure relativement rigide et perd la conformabilité que l'on souhaite obtenir de par la mise en œuvre d'une technologie souple. Au demeurant, le réseau de diodes LED n'est pas continu, de sorte que l'homogénéité de la lumière au niveau du corps du nourrisson n'est pas satisfaisante. Enfin, la présence de telles diodes LED directement au sein de la couverture engendre un risque d'échauffement, nécessitant l'intégration de capteurs de température et, lors sa mise en œuvre, le monitoring du nourrisson.

Afin d'optimiser la conformabilité d'un tel dispositif de photothérapie, il a été proposé, par exemple dans le document CN 106861048, la mise en œuvre d'un dispositif de photothérapie intégrant une structure textile émettant de la lumière obtenue par tissage de fibres optiques à émission latérale.

Il peut cependant être observé que ce textile est relativement complexe à réaliser et nécessite, afin d'obtenir l'homogénéité de l'éclairement souhaitée, de mettre en œuvre des fibres optiques chargées de nano-charges de dioxyde de titane, sur lesquelles on vient ajouter un nano-coating d'un microcristal par traitement corona. Ces différentes étapes de traitement rendent un tel dispositif onéreux, le rendant dès lors peu accessible aux centres de soins. En outre, le mode de réalisation d'un tel dispositif rigidifie le textile lumineux, antinomique avec les résultats recherchés.

On a également proposé dans le document WO 2013/068518, une enveloppe souple multicouche diffuseuse de lumière, réalisée sur une base textile tissée avec fibres optiques à émission latérale. Si, incontestablement, ce dispositif présente une souplesse améliorée, et donc permet d'optimiser le confort du nouveau-né, il nécessite cependant l'inclusion de microcapsules à changement de phase destinées à réguler la température, surenchérissant les coûts de fabrication. En outre, le dispositif décrit dans ce document ne permet pas de s'affranchir des risques d'enfouissement du nouveau-né au sein de l'enveloppe, avec les risques d'étouffement à la clé.

Le document WO 2016/012732 décrit un dispositif textile souple réalisé par tissage avec fibres optiques à émission latérale. Si là encore, ce dispositif présente une avancée en termes de souplesse et corollairement de confort du nourrisson, le problème de la gestion de l'échauffement inhérent à la diffusion de lumière demeure entier. Il est en effet préconisé, pour lutter contre cet échauffement, la réalisation d'un volume défini par la disposition de parois latérales avec des ouvertures pour assurer une circulation d'air, mais qui corollairement engendre une fuite de lumière, et qui là encore, nécessite une vigilance importante vis-à-vis du risque d'hyperthermie.

Le document US 2009/030489 divulgue un sac de couchage adapté pour fournir une photothérapie.

Le document FR 3 029 944 décrit une structure textile tricotée mettant en oeuvre des fibres optiques intégrées dans la structure textile, afin notamment de diffuser de la lumière par courbure desdites fibres optiques, mieux connue sous l'expression « macro-bending ».

En résumé, il n'existe pas à ce jour de dispositif souple de traitement par photothérapie alliant notamment le confort et le bien-être du patient, et en l'espèce du nourrisson, avec l'efficacité du traitement dans des conditions de sécurité optimisées.

### EXPOSE DE L'INVENTION

L'invention s'attache à répondre à ces différentes exigences.

Elle concerne un dispositif du type souple pour le traitement de patients par photothérapie.

Elle vise à répondre aux exigences suivantes :
- réglage de la quantité de lumière émise ;
- homogénéité de la lumière émise sur tout le corps du patient ;
- limitation voire annulation de la chaleur générée par la structure ;
- optimisation du confort du patient ;
- optimisation du contact mère-enfant ;
- intégration d'un système anti-enfouissement du nouveau-né au sein du dispositif ;
- limitation voire annulation de toute lumière émise en direction de l'extérieur du dispositif ;
- possibilité d'extraire le nouveau-né très facilement, notamment sans effort et en un seul geste et très rapidement en cas de détresse médicale ;
- un système garantissant un haut niveau d'hygiène afin de répondre aux risques de maladies nosocomiales qui se développent dans les hôpitaux.

A cet effet, l'invention propose un dispositif pour le traitement de patients par photothérapie comprenant :
- une première partie à usage multiple, constituée d'une structure textile souple, comprenant des générateurs de rayonnement réalisés par tricotage et intégrant des fibres optiques, dont au moins l'une des extrémités est connectée à une source de lumière, lesdites fibres optiques étant intégrées dans les mailles du tricot, ou positionnées en trames partielles dans le tricot de telle sorte à émettre de la lumière dans la longueur d'onde d'intérêt en raison du dépassement de leur angle de courbure limite, selon la technologie dite du « macro-bending » ;
- une seconde partie à usage unique, associée de manière amovible à ladite première partie, et destinée à venir en contact du patient traité, et apte à diffuser en direction de ce dernier la lumière émise par ladite première partie.

En d'autres termes, l'invention consiste, en premier lieu, à prévoir deux structures distinctes souples, associées de manière réversible l'une à l'autre :
- l'une destinée à générer le rayonnement lumineux dans la gamme de longueurs d'onde souhaitée, et intégrant de fait au moins un générateur de rayonnement, dont la structure est telle qu'elle permet d'obtenir une homogénéité de l'éclairement du patient sur une partie importante de son corps ;
- et l'autre destinée à être à usage unique, et donc typiquement constituée d'une housse jetable, ce dans l'objectif de conserver intact ledit dispositif de génération de rayonnement, facilement désolidarisable de ce dernier, et ainsi favoriser l'usage dudit dispositif dans des conditions d'hygiène acceptables dans le domaine médical considéré.

Selon l'invention, la structure de la première partie à usage multiple définit une surface lumineuse permettant d'illuminer à minium 60% de la surface du corps du patient.

Selon un autre aspect de l'invention, les fibres optiques mises en œuvre au sein de ladite première partie à usage multiple, c'est-à-dire de la structure générant le rayonnement, sont tricotées de telle sorte que lesdites fibres optiques sont courbées au-delà de l'angle de courbure limite desdites fibres. Cet angle de courbure limite dépend intrinsèquement du matériau constituant la fibre optique, ainsi que du diamètre de cette dernière. Ainsi, pour une fibre optique considérée, l'émission de lumière s'effectue dans le plan de courbure et est dépendante de la contrainte appliquée à ladite fibre. En d'autres termes, et contrairement aux dispositifs de l'art antérieur, en raison de l'absence de sortie directe de la lumière, il n'y a pas d'échauffement, optimisant en conséquence de manière importante la sécurité du dispositif et simplifiant son utilisation puisqu'il n'est plus nécessaire de mettre en place le monitoring du nourrisson, et de manière générale du patient pendant la phase de traitement. Afin de disposer d'une intensité de diffusion constante avec cette technologie, au demeurant recherchée dans le cadre de la présente invention, l'angle de courbure limite desdites fibres optiques doit pouvoir être conservé lors de la mise en œuvre du textile les intégrant.

Cette technologie a été décrite, notamment dans le document WO 2016/097524. En l'espèce, comme déjà dit, les fibres optiques sont courbées au-delà de leur angle de courbure limite, le tricot dans lequel elles s'intègrent étant obtenu par technologie à mailles jetées type chaîne ou Rachel ou crochet. Le nombre d'armures du tricot permet de maintenir la courbe souhaitée des fibres optiques, toute en conférant une certaine souplesse et une certaine extensibilité de la structure, et par voie de conséquence permet de maîtriser la quantité et la qualité de lumière émise. En outre, cette technologie permet de mettre en œuvre des fibres optiques de diamètre plus réduit, conférant en conséquence au textile dans lequel elles sont intégrées une souplesse accrue, et au dispositif un degré de conformation plus élevé.

Selon l'invention, la première partie à usage multiple intégrant les générateurs de rayonnement, comprend une paroi dorsale et une paroi ventrale, reliées l'une à l'autre au niveau de leur partie inférieure respective, définissant ainsi une charnière.

Chacune de ces parois dorsale et ventrale présente au niveau de leur extrémité supérieure une découpe apte à définir une ouverture de type encolure, destinée à permettre l'émergence de la tête du patient hors du dispositif tout en limitant la fuite de lumière émise en direction de la tête du patient.

Selon l'invention, la seconde partie à usage unique est constituée d'une double poche, dont les dimensions sont sensiblement égales ou légèrement supérieures à celles des parois dorsale et ventrale de ladite première partie à usage multiple, chacune des deux poches étant destinée à venir s'enfiler par-dessus lesdites parois ventrale et dorsale, d'où le terme de housse précité ; la face interne de cette seconde partie à usage unique, destinée à venir au contact du patient, est continue ; en d'autres termes, les deux poches sont solidarisées l'une à l'autre par ladite face interne continue.

Par ailleurs et selon l'invention, la face interne de la seconde partie à usage unique, et destinée à venir au contact du dos du patient, est munie d'un système anti-enfouissement du nouveau-né, c'est-à-dire d'un moyen apte à s'opposer à la descente dudit nouveau-né le dispositif de l'invention, dont le risque évident réside dans l'étouffement du nourrisson. Ce système est composé d'une bande destinée à passer entre les jambes du nourrisson, dont l'extrémité libre est solidarisable réversiblement audit nourrisson, typiquement sur la couche dont ce dernier est traditionnellement muni. Il en résulte, outre la fonction anti-enfouissement recherchée, une grande simplicité de positionnement du nourrisson à l'intérieur du dispositif de l'invention quelle que soit la taille de ce dernier, et corollairement, une grande facilité d'extraction du nouveau-né hors du dispositif. Typiquement ce mode de solidarisation réversible peut être constitué d'un système boucles et crochets, mieux connu sous la marque déposée VELCRO. Avantageusement, les extrémités libres de l'une des deux poches constitutives de la seconde partie à usage unique, sont munies de moyens de solidarisation réversible du type rabat, aptes à coopérer avec l'autre poche, et destinés à limiter la fuite de lumière émise par le dispositif en direction de la tête du patient.

On l'aura bien compris, l'invention vise également à limiter, voire supprimer toute fuite de lumière en dehors du volume destiné à recevoir le patient et en l'espèce le nourrisson.

### BREVE DESCRIPTION DES FIGURES

La manière dont l'invention peut être réalisée, et les avantages qui en découlent, ressortiront mieux de l'exemple de réalisation qui suit, donné à titre indicatif et non limitatif à l'appui des figures annexées.
La figure 1 est une représentation schématique en perspective du dispositif de l'invention.
La figure 2 est une représentation schématique de la première partie à usage multiple du dispositif de l'invention en position ouverte.
La figure 3 est une représentation schématique en section partielle du dispositif de l'invention en position semi ouverte.
La figure 4 illustre schématiquement le dispositif de l'invention avec le moyen anti-enfouissement.

### DESCRIPTION DETAILLEE DE L'INVENTION

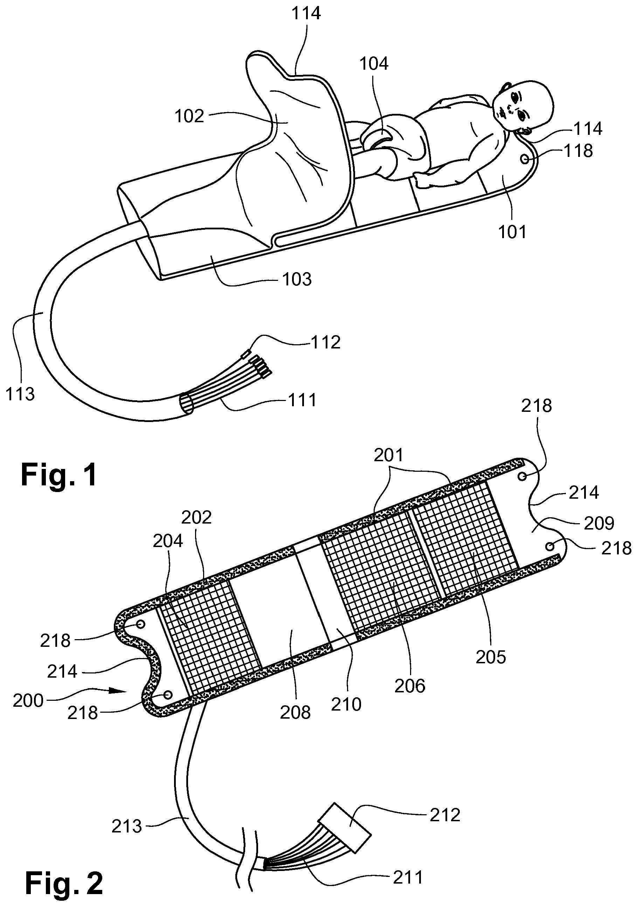

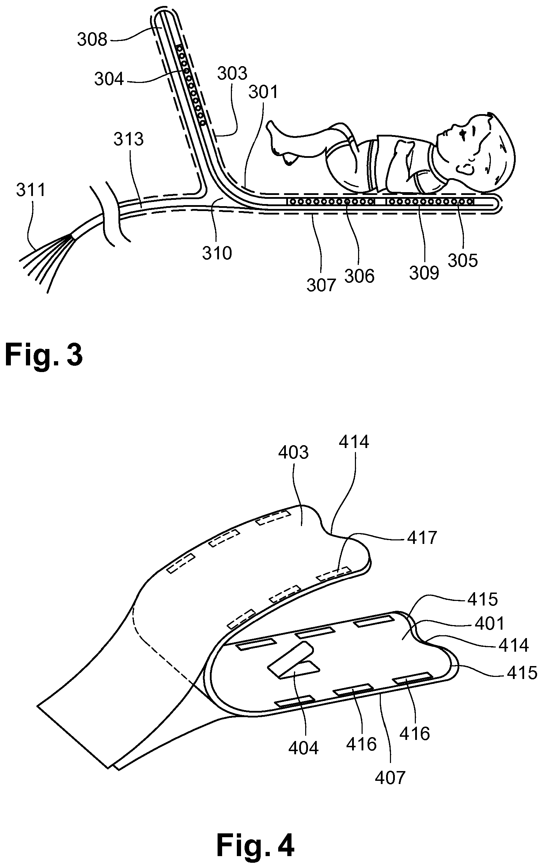

On a donc schématiquement représenté au sein des figures le dispositif souple de traitement par photothérapie de l'invention. Fondamentalement, celui-ci se présente en quelque sorte sous la forme d'une turbulette ou grenouillère, puisque principalement destiné à être utilisé pour des nouveau-nés. Il est constitué de deux structures souples, réversiblement associées l'une à l'autre :
- une première structure dite à usage multiple (200), et dans les faits intégrant trois générateurs de lumière ou de rayonnement (204, 205, 206 ; 304, 305, 306), décrit plus en détail en relation avec les figures 2 et 3 ;
- et une seconde structure « jetable », donc à usage unique (103 ; 303, 307 ; 403, 407), ayant notamment une finalité hygiénique d'une part, et de confort du patient d'autre part. Typiquement, il s'agit d'une housse, puisqu'ayant vocation à protéger ladite première structure, notamment contre les selles et urines du nouveau-né.

Le générateur de lumière est constitué d'au moins deux structures textiles tricotées (204, 205, 206 ; 304, 305, 306), cousues sur une membrane (208, 209 ; 308, 309), définissant une paroi dorsale (209, 205, 206 ; 309, 305, 306) et une paroi ventrale (208, 204 ; 308, 304) reliées l'une à l'autre au niveau de leur extrémité inférieure respective et constituant ainsi une charnière (210, 310).

Le générateur de lumière est, comme déjà dit, constitué d'un tricot mettant en œuvre la technologie à mailles jetées sur métier Rachel, chaîne ou crochet, intégrant en son sein des fibres optiques selon une armure telle, que leur courbure dépasse leur angle de courbure limite, de telle sorte à émettre ladite lumière dans le plan de la courbure. A cet effet, les fibres optiques sont, soit totalement intégrées dans la maille du tricot afin d'obtenir une lumière ponctuelle et intense, soit positionnées en trame partielle selon un pas constant, afin d'obtenir une lumière homogène et d'intensité sensiblement uniforme sur toute la surface dudit textile.

Le tricot intégrant les fibres optiques est typiquement réalisé en polyester. Cependant, tout autre matériau pourrait être mis en œuvre, tel que le lin, le coton, le polyamide, etc.

La membrane (208, 209 ; 308, 309) sur laquelle ces générateurs de lumière sont cousus, est par exemple, un bi-couche comportant :
- une couche interne constituée d'une membrane fine, blanche, brillante ou mat, imperméable et respirante, destinée tout d'abord à orienter la lumière générée par le générateur de lumière en direction du patient, et à empêcher la fuite de la lumière en direction de l'extérieur du dispositif, telle que par exemple une membrane imper-respirante d'une épaisseur typique comprise entre 50 et 400 micromètres ; cette membrane peut par exemple être réalisée en polyuréthane ;
- et une couche externe, avantageusement de couleur foncée, de telle sorte à bloquer le peu de lumière susceptible de diffuser à travers la couche interne, et par exemple constituée d'un textile en polyester d'une densité comprise entre 75 et 250 g/m².

La couche interne répond avantageusement au standard Oekotex 100 classe 1 ou équivalent **https://www.oeko-tex.com/fr/business/certifications and services/ ots 100/ots 100 product classes/ots 100 product classes.html** ;Classe de produits I : textiles et jouets textiles pour les bébés et enfants jusqu'à leurs 3 ans révolus, par ex. sous-vêtements, barboteuses, draps, articles de literie, animaux en tissu etc.

Les parois dorsale et ventrale de cette membrane sont réunies l'une à l'autre par confection au niveau d'une zone charnière (210, 310).

Les fibres optiques intégrées dans les structure textiles tricotés constitutives des générateurs de rayonnement sont rassemblées dans un faisceau (111, 211, 311) de fibres optiques, chacune desdites fibres optiques étant connectée (112, 212) à une source de lumière de type LED, OLED ou laser (non représentée), dont la longueur d'onde correspond aux dernières recommandations de spectre pour le traitement de la jaunisse ; actuellement, ce spectre est typiquement compris entre 420 et 500 nanomètres, mais l'invention est susceptible de s'adapter à d'autres recommandations médicales.

Comme on peut l'observer sur les figures 2 et 3, et afin de garantir l'absence de tout contact entre les fibres optiques libres, c'est-à-dire non intégrés dans les générateurs de rayonnement, et le nouveau-né, le faisceau desdites fibres optiques est entouré d'un manchon (113, 213, 313) qui aboutit au niveau de la zone de liaison ou charnière (210, 310) de la paroi dorsale et de la paroi ventrale définissant ladite première structure à usage multiple.

Les extrémités supérieures libres des parois, respectivement dorsale et ventrale, de ladite première structure à usage multiple présentent une découpe (214) définissant, lorsqu'elles sont solidarisées réversiblement l'une à l'autre par le biais de ladite seconde structure à usage unique (voir ci-après) une encolure de laquelle est susceptible d'émerger la tête du nourrisson. A cet effet, la structure à usage unique présente des découpes équivalentes (114, 414) et sont munies d'un système de fixation par boucles et crochets (de type Velcro^{®}), de telle sorte d'une part, à empêcher la tête du nourrisson de pénétrer dans l'espace défini par lesdites parois dorsale et ventrale, évitant le risque d'enfouissement du nourrisson au sein de cet espace, et d'autre part, à empêcher la lumière générée dans ledit espace à sortir hors de ce dernier, et corollairement à illuminer la tête, et donc notamment les yeux du nourrisson.

Selon l'invention, cette première structure ou partie à usage multiple est destinée à recevoir de manière amovible une seconde structure à usage unique, typiquement jetable. Celle-ci définit deux poches (303, 307) destinées à venir s'enfiler au niveau des deux parois dorsale et ventrale de ladite première partie à usage multiple, et donc de dimensions sensiblement correspondantes en largeur à tout le moins et au jeu près des parois dorsale et ventrale de la structure à usage multiple afin de faciliter l'enfilage dans lesdites poches. Cependant, et ainsi qu'on peut bien l'observer sur les figures 1, 3 et 4, la face interne (101, 301, 401) de cette partie jetable est continue, afin de préserver la partie à usage multiple de toutes salissures (urine, selles). Il est donc défini une poche dorsale (307) et une poche ventrale (303). En raison de la mise en œuvre de cette seconde partie jetable à usage unique, on optimise les conditions d'hygiène et on s'affranchit des problèmes de nettoyage et de risque de contamination de la structure essentielle du dispositif, c'est-à-dire du générateur de lumière.

Cette partie jetable ou housse est typiquement constituée d'un multi-matériau, intégrant typiquement un non-tissé et une couche imper-respirante ayant globalement des propriétés de passage de la lumière de 80 à 100% dans son intégralité, permettant à la lumière émise par les générateurs de lumière de diffuser à travers en direction du nourrisson.

Ce multicouche est typiquement un bicouche, comportant respectivement :
- une face destinée à venir en contact avec la peau du patient, donc douce et absorbante, par exemple de type non-tissé ou matière absorbante et non-irritante par exemple réalisée à base de micro-fibres PET (polyéthylène téréphtalate) ou en matière naturelle ; et
- une face en contact avec les générateurs de lumière, imperméable afin de protéger ces derniers d'éventuelles souillures (urines, selles) et préserver les conditions d'hygiène du dispositif de l'invention par exemple une membrane imper-respirante transparente; cette face est par exemple constituée d'une membrane en polyuréthane.

Ces deux faces peuvent être laminées entre elles par thermocollage ou par un réseau de colle. L'ensemble ainsi constitué permet une bonne transmission lumineuse de l'ordre de 85 à 98% en direction du nourrisson.

Cette partie jetable présente un moyen anti-enfouissement constitué d'une bande (104, 404) émanant de la partie dorsale, et apte à venir se solidariser réversiblement, typiquement sur la couche dont est muni le nourrisson au moyen d'un système boucles/crochets. On conçoit de fait la grande facilité de positionnement du nourrisson sur le dos sur la partie dorsale de ladite première partie à usage multiple revêtue de la housse jetable, puis positionnement de cette bande sur la couche du nourrisson à la hauteur souhaitée pour permettre à la tête de ce dernier d'émerger hors du dispositif de l'invention.

En outre, les extrémités libres de ladite partie jetable présentent une découpe destinée à définir l'encolure, de manière analogue à la découpe réalisée à l'extrémité supérieure des parois dorsale et ventrale de la structure à usage multiple, et dans le même but, à savoir l'émergence de la tête du nourrisson hors du dispositif de l'invention (cf. ci-dessus).

Par ailleurs, les extrémités libres de la poche dorsale de la partie jetable présentent un rabat (415) muni sur sa face interne d'un système de solidarisation réversible du type boucles et crochets, destiné à coopérer avec les extrémités libres de la face externe de la poche ventrale, dont la matière en non-tissé permet l'adhérence avec le système boucles et crochets. On évite ainsi, au niveau de l'encolure ainsi définie, toute sortie de lumière, permettant de s'affranchir pour le nourrisson du port de lunettes ou d'un bandeau anti-lumière. En d'autres termes, la lumière est confinée à l'intérieur du dispositif de l'invention, lorsque celui-ci est en place autour du nourrisson.

Cette conception de la partie jetable ou à usage unique offre donc une garantie très élevée contre le risque d'enfouissement du bébé, qui ne peut ainsi pas descendre dans le dispositif grâce à une double sécurité résultant d'une part, de la bande de support qui lui passe entre les jambes et solidarisée sur la couche par le système boucles crochets, et d'autre part, par la fermeture des rabats ménagés à l'extrémité supérieure de la structure à usage unique, qui une fois positionnés, ne permettent pas le passage de la tête du nourrisson à l'intérieur du dispositif.

La partie jetable à usage unique comporte en outre, soit sur sa partie dorsale, soit sur sa partie ventrale, un système de fixation réversible de type boucles crochets (416, 417) se présentant sous la forme de bandes ménagées le long des deux bords latéraux de la partie considérée, et destinées à coopérer avec la face en non-tissé de l'autre partie. Ce faisant, on assure par ce biais, en coopération avec le système d'encolure décrit précédemment, le confinement de la lumière à l'intérieur du dispositif de l'invention. En outre, en raison de ce mode de fixation réversible, l'extraction du nourrisson hors dudit dispositif en cas d'urgence est rendue aisée.

Par ailleurs la seconde partie à usage unique peut avantageusement comporter un émetteur de type RFID, « radio-étiquette », ou équivalent, susceptible d'être activée par la source de lumière intégrant alors, selon une technologie bien connue, un émetteur-récepteur adapté à la fréquence de l'étiquette.

Cette solution technique permet de contrôler la présence ou le changement systématique de la partie à usage unique, garantissant d'une part, une sécurité d'usage, et d'autre part, l'hygiène de l'ensemble du dispositif pour le patient.

L'automatisme alors intégré dans le dispositif, garantit la non-activation de la source de lumière en l'absence de la partie jetable ou lorsque ladite partie jetable a déjà servi, afin de garantir l'hygiène par sa présence et d'éviter tout risque de contamination entre les nourrissons.

Alternativement tous les systèmes boucles crochets décrit ci-dessus peuvent être remplacés par d'autres moyens de fixation de type boutons pressions ou tout autre système de fixation rapide et simple à libérer en en seul mouvement, afin de permettre un soin en cas d'urgence vitale pour le nourrisson.

Avantageusement, la partie jetable est solidarisée réversiblement à la partie à usage multiple au moyen de boutons pressions (118, 218), positionnées aux extrémités respectives des parties dorsales et ventrales de la partie jetable et de la partie à usage multiple.

On conçoit tout l'intérêt du dispositif de traitement par photothérapie conforme à l'invention.

Tout d'abord, il répond à toutes les exigences médicales en termes :
- d'irradiance : efficacité du traitement ;
- de sécurité du nourrisson : pas de chaleur générée, présence d'un système anti-enfouissement sans pour autant entraver la facilité d'extraction du nourrisson du dispositif en un geste simple et rapide en cas d'urgence ;
- d'absence de fuite de lumière, évitant ainsi l'inconfort du port de lunettes ou de bandeau sur les yeux ;
- d'hygiène de par la présence de la seconde partie à usage unique, typiquement constitutive d'une housse jetable ;
- de double système sécurisé concernant l'anti-enfouissement et la libération ultra-rapide du nourrisson par rapport au dispositif, afin de permettre des soins en cas d'urgence vitale quelle qu'en soit la cause.

Le dispositif de l'invention permet en outre d'optimiser de manière significative le confort et le bien-être du nourrisson :
- en effet, bien que relié à une source de lumière, le dispositif permet de porter l'enfant et donc d'assurer un contact parent-enfant ;
- il est envisageable d'utiliser ce dispositif en traitement à domicile, afin ainsi d'éviter les hospitalisations dans les services pédiatriques et la séparation de la mère et de l'enfant, puisqu'aussi bien, il n'est plus nécessaire de monitorer l'enfant pendant les phases de traitement ;
- la durée de traitement peut être optimisée par rapport à la mise en œuvre d'un tunnel classique puisqu'il devient possible, par exemple, de donner à manger à l'enfant pendant le traitement.

## Revendications

1. Dispositif pour le traitement de patients par photothérapie, comprenant une première partie à usage multiple (200, 308, 309), constituée d'une structure textile souple comprenant des générateurs de rayonnement (204, 205, 206 ; 304, 305, 306) réalisés par tricotage et intégrant des fibres optiques (111, 211, 311), dont au moins l'une des extrémités est connectée à une source de lumière, lesdites fibres optiques étant intégrées dans les mailles du tricot ou positionnées en trames partielles dans le tricot, de telle sorte à émettre de la lumière en raison du dépassement de leur angle de courbure limite, selon la technologie dite du « macro bending » ; et une seconde partie à usage unique (103 ; 303, 307 ; 403, 407), associée de manière amovible à ladite première partie, destinée à venir au contact du patient traité et apte à diffuser en direction de ce dernier la lumière émise par ladite première partie,
la première partie à usage multiple comprenant une paroi dorsale (209 ; 309) et une paroi ventrale (208 ; 308) reliées l'une à l'autre au niveau de leur partie inférieure respective formant charnière 210 ; 310), et présentant chacune au niveau de leur extrémité supérieure une découpe (214) apte à définir une ouverture de type encolure, destinée à permettre l'émergence de la tête du patient hors dudit dispositif, tout en limitant la fuite de la lumière émise par ladite première partie en direction de la tête, parois dorsale et ventrale sur lesquels sont rapportés, notamment par couture les générateurs de lumière ;
la seconde partie à usage unique étant constituée d'une double poche (303, 307), chacune des deux poches étant destinées à venir s'enfiler par-dessus les parois ventrale et dorsale de ladite première partie à usage multiple, la face interne (101, 301, 401) de ladite seconde partie, destinée à venir au contact du patient, étant continue.

2. Dispositif pour le traitement de patients par photothérapie selon la revendication 1, **caractérisé en ce que** la face interne de la seconde partie à usage unique, destinée à venir au contact du dos du patient est munie d'un système anti-enfouissement composé d'une bande (104 ; 404) destinée à passer entre les jambes du patient, et dont l'extrémité libre est solidarisable réversiblement audit patient.

3. Dispositif pour le traitement de patients par photothérapie selon l'une des revendications 1 et 2, **caractérisé en ce que** les extrémités libres de l'une des deux poches de la seconde partie à usage unique sont munies de moyens de solidarisations réversibles aptes à coopérer avec l'autre poche, et destinés à limiter la fuite de la lumière émise par ledit dispositif en direction de la tête du patient.

4. Dispositif pour le traitement de patients par photothérapie selon l'une des revendications 1 à 3, **caractérisé en ce que** la première partie à usage unique est réalisée à base d'une membrane bi-couche laminée répondant au standard Oekotex 100 classe 1 ou équivalent.

5. Dispositif pour le traitement de patients par photothérapie selon la revendication 4, **caractérisé en ce que** la couche interne de la partie à usage multiple est une membrane fine blanche imperméable respirante et apte à orienter la lumière en direction du patient et empêcher la fuite vers l'extérieur du dispositif, et **en ce que** la couche externe de ladite partie à usage multiple est de couleur foncée afin de bloquer la lumière susceptible de passer à travers la membrane interne en direction de l'extérieur dudit dispositif.

6. Dispositif pour le traitement de patients par photothérapie selon l'une des revendications 1 à 5, **caractérisé en ce que** la seconde partie à usage unique est constitué d'un multi-matériau, et notamment d'un bicouche, dont la face interne destinée à venir en contact du patient est avantageusement constituée d'un non-tissé et laisse diffuser la lumière émise par ladite première partie à usage multiple, et dont la face externe en contact avec ladite première partie à usage multiple est imperméable.

7. Dispositif pour le traitement de patients par photothérapie selon l'une des revendications 1 à 6, **caractérisé en ce que** la seconde partie à usage unique intègre un émetteur de type RFID, activable par la source de lumière à laquelle sont connectées les fibres optiques, et destiné à contrôler la présence de ladite partie à usage unique sur le dispositif et/ou l'occurrence de l'utilisation antérieure de ladite partie à usage unique en vue de son changement.

8. Dispositif pour le traitement de patients par photothérapie selon l'une des revendications 1 à 7, **caractérisé en ce que** la source de lumière est constituée d'une diode LED, OLED ou un laser émettant une lumière bleue dont la longueur d'onde est comprise entre 420 et 500 nanomètres.

9. Dispositif pour le traitement de patients par photothérapie selon l'une des revendications 1 à 8, **caractérisé en ce que** la structure de la première partie à usage multiple définit une surface lumineuse permettant d'illuminer à minium 60% de la surface du corps du patient.

## Patentansprüche

1. Vorrichtung zur Behandlung von Patienten durch Phototherapie, die ein erstes mehrfach verwendbares Teil (200, 308, 309) umfasst, das aus einer biegsamen Textilstruktur mit Strahlungsgeneratoren (204, 205, 206, 304, 305, 306) besteht, die durch Stricken hergestellt sind und Glasfasern (111, 211, 311) enthalten, wobei mindestens eines der Enden mit einer Lichtquelle verbunden ist, die Glasfasern in die Maschen des Gestricks integriert oder in Schussteilen im Gestrick positioniert sind, sodass sie aufgrund der Überschreitung ihres maximalen Biegungswinkels nach der sogenannten "Makro-Biegung" Licht emittieren; und
ein zweites einmal verwendbares Teil (103, 303, 307, 403, 407), das abnehmbar mit dem ersten Teil verbunden ist und mit dem zu behandelnden Patienten in Kontakt treten soll und das vom ersten Teil ausgestahlte Licht in Richtung des Patienten streuen kann,
wobei der erste mehrfach verwendbare Teil eine Rückenwand (209, 309) und eine Bauchwand (208, 308) umfasst, die an ihrem jeweiligen unteren Teil miteinander verbunden sind und ein Scharnier (210; 310) bilden, und jeweils an ihrem oberen Ende einen Ausschnitt (214) aufweisen, der eine halsausschnittartige Öffnung bildet, durch die der Kopf des Patienten aus der Vorrichtung ausragt und welche den Asutritt des vom ersten Teil ausgestrahlten Lichts in Richtung des Kopfes und der Rücken- und Bauchwände in Grenzen hält, an denen die Lichtgeneratoren vor allem durch Nähte befestigt sind;
wobei der zweite einmal verwendbare Teil aus einer Doppeltasche (303, 307) besteht und jede der beiden Taschen über die Bauch- und Rückenwände des ersten mehrfach verwendbaren Teils gestülpt werden kann, und die Innenseite (101, 301, 401) des zweiten Teils, die durchgehend ist, mit dem Patienten in Kontakt treten kann.

2. Vorrichtung zur Behandlung von Patienten durch Phototherapie nach Anspruch 1, **dadurch gekennzeichnet, dass** die Innenseite des zweiten einmalig verwendbaren Teils, das mit dem Rücken des Patienten in Kontakt tritt, mit einem System zum Schutz gegen Eindringung versehen ist, das aus einer Binde (104, 404) besteht, die zwischen den Beinen des Patienten hindurchgeführt wird und dessen freies Ende reversibel mit dem Patienten verbunden werden kann.

3. Vorrichtung zur Behandlung von Patienten durch Phototherapie nach einem der Ansprüche 1 und 2, **dadurch gekennzeichnet, dass** die freien Enden einer der beiden Taschen des zweiten einmalig verwendbaren Teils mit reversiblen Befestigungsmitteln versehen sind, die mit der anderen Tasche zusammenwirken können und den Austritt des von der Vorrichtung emittierten Lichts in Richtung des Kopfes des Patienten in Grenzen halten sollen.

4. Vorrichtung zur Behandlung von Patienten durch Phototherapie nach einem der Ansprüche 1 bis 3, **dadurch gekennzeichnet, dass** der erste einmalig verwendbare Teil auf der Basis einer laminierten Doppelmembran hergestellt ist, die dem Standard Ökotex 100 Klasse 1 oder einem gleichwertigen Standard entspricht.

5. Vorrichtung zur Behandlung von Patienten durch Phototherapie nach Anspruch 4, **dadurch gekennzeichnet, dass** die innere Schicht des mehrfach verwendbaren Teils eine dünne, weiße, undurchlässige, atmungsaktive Membran ist, die das Licht in Richtung des Patienten lenken und verhindern kann, dass es aus der Vorrichtung austritt, und dass die äußere Schicht des mehrfach verwendbaren Teils eine dunkle Farbe aufweist, um Licht abzublocken, das durch die innere Membran aus der Vorrichtung nach außen austreten könnte.

6. Vorrichtung zur Behandlung von Patienten durch Phototherapie nach einem der Ansprüche 1 bis 5, **dadurch gekennzeichnet, dass** der zweite einmalig verwendbare Teil aus einem Multimaterial, insbesondere einer Doppelschicht, besteht, deren Innenseite, die Kontakt mit dem Patienten aufnehmen soll, vorzugsweise aus einem Vliesstoff besteht und das vom ersten mehrfach verwendbaren Teil ausgestrahlte Licht durchlässt, und deren Außenseite, die mit dem ersten mehrfach verwendbaren Teil in Kontakt steht, undurchlässig ist.

7. Vorrichtung zur Behandlung von Patienten durch Phototherapie nach einem der Ansprüche 1 bis 6, **dadurch gekennzeichnet, dass** der zweite einmalig verwendbare Teil einen RFID-Sender enthält, der durch die Lichtquelle, an die die Glasfasern angeschlossen sind, aktiviert werden kann und der dazu bestimmt ist, zu kontrollieren, ob das einmalig verwendbare Teil an der Vorrichtung vorhanden ist und/oder ob das einmalig verwendbare Teil vorher verwendet wurde und ausgetauscht werden muss.

8. Vorrichtung zur Behandlung von Patienten durch Phototherapie nach einem der Ansprüche 1 bis 7, **dadurch gekennzeichnet, dass** die Lichtquelle aus einer LED-, OLED- oder Laserdiode besteht, die blaues Licht mit einer Wellenlänge zwischen **420** und 500 Nanometern emittiert.

9. Vorrichtung zur Behandlung von Patienten durch Phototherapie nach einem der Ansprüche 1 bis 8, **dadurch gekennzeichnet, dass** mit der Struktur des ersten mehrfach verwendbaren Teils eine Leuchtfläche festgelegt wird, mit der mindestens 60 % der Körperoberfläche des Patienten beleuchtet werden kann.

## Claims

1. Device for the treatment of patients by phototherapy, comprising a first multiple-use portion (200, 308, 309), formed of a flexible textile structure, comprising radiation generators (204, 205, 206; 304, 305, 306) formed by knitting and integrating optical fibers (111, 211, 311), at least one end thereof being connected to a light source, said optical fibers being integrated in the knit stitches, or positioned in partial wefts in the knit, to emit light due to the exceeding of their limiting bending angle, according to the so-called "macro-bending" technology; and
a second single-use portion (103; 303, 307; 403, 407), removably associated with said first portion, intended to come into contact with the treated patient, and capable of diffusing towards the latter the light emitted by said first portion;
the first multiple-use portion comprising a dorsal wall (209; 309) and a ventral wall (208; 308) connected to each other at the level of their respective hinge-forming lower portion (210; 310) and each comprising at the level of their upper end a cutout (214) capable of defining a neck-line type opening, intended to enable the patient's head to emerge out of said device, while limiting leaks of the light emitted by said first portion towards the head, on which dorsal and ventral walls the light generators are arranged, particularly by sewing ;
the second single-use portion being formed of a double pocket (303, 307), each of the two pockets being intended to slip on the ventral and dorsal walls of said first multiple-use portion, the inner surface (101, 301, 401) of said second portion, intended to come into contact with the patient, being continuous.

2. Device for the treatment of patients by phototherapy according to claim 1, **characterized in that** the inner surface of the second single-use portion, intended to come into contact with the patient's back, is provided with a burying prevention system formed of a band (104; 404) intended to pass between the patient's legs, and having its free end reversibly attachable to said patient.

3. Device for the treatment of patients by phototherapy according to one of claims 1 and 2, **characterized in that** the free ends of one of the two pockets of the second single-use portion are provided with reversible attachment means capable of cooperating with the other pocket, and intended to limit leaks of the light emitted by said device towards the patient's head.

4. Device for the treatment of patients by phototherapy according to one of claims 1 to 3, **characterized in that** the first single-use portion is formed from a laminated bilayer membrane complying with the Oekotex 100 class-1 standard or the like.

5. Device for the treatment of patients by phototherapy according to claim 4, **characterized in that** the inner layer of the multiple-use portion is a thin white breathable impermeable membrane capable of orienting the light towards the patient and of preventing leaks to the outside of the device, and **in that** the external layer of said multiple-use portion is dark-colored to block the light likely to pass through the inner membrane towards the outside of said device.

6. Device for the treatment of patients by phototherapy according to one of claims 1 to 5, **characterized in that** the second single-use portion is made of a multi-material, and particularly of a bilayer, having its inner surface intended to come into contact with the patient advantageously made of a nonwoven and which lets the light emitted by said first multiple-use portion diffuse, the outer surface thereof which is in contact with said first multiple-use portion being impermeable.

7. Device for the treatment of patients by phototherapy according to one of claims 1 to 6, **characterized in that** the second single-use portion integrates a RFID-type emitter, capable of being activated by the light source to which the optical fibers are connected, and intended to control the presence of said single-use portion on the device and/or the occurrence of the prior use of said single-use portion for its change.

8. Device for the treatment of patients by phototherapy according to one of claims 1 to 7, **characterized in that** the light source is formed of a LED, OLED diode or of a laser emitting blue light having a wavelength in the range from 420 to 500 nanometers.

9. Device for the treatment of patients by phototherapy according to one of claims 1 to 8, **characterized in that** the structure of the first multiple-use portion defines a light-emitting surface enabling to illuminate at least 60% of the surface area of the patient's body.
